(19) **Europäisches Patentamt / European Patent Office / Office européen des brevets**

(11) **EP 4 624 024 A1**

(12) **EUROPEAN PATENT APPLICATION**

(43) Date of publication:
01.10.2025 Bulletin 2025/40

(51) International Patent Classification (IPC):
*B01D 53/22* (2006.01)   *B01D 69/14* (2006.01)
*C07C 7/144* (2006.01)

(21) Application number: 24166925.8

(22) Date of filing: 27.03.2024

(52) Cooperative Patent Classification (CPC):
(C-Sets available)
**B01D 53/228; B01D 69/14; B01D 69/141;
B01D 69/148; C07C 7/005; C07C 7/144;**
B01D 2256/24; B01D 2257/102; B01D 2257/108;
B01D 2257/7022; B01D 2257/7025;
B01D 2259/4516; B01D 2317/00; B01D 2317/022;
B01D 2317/025                                    (Cont.)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: UniSieve Ltd
8045 Zürich (CH)

(72) Inventors:
• SCHNEIDER, Elia
8045 Zürich (CH)
• HESS, Samuel
8045 Zürich (CH)
• JEHANNIN, Marie
8045 Zürich (CH)

(74) Representative: E. Blum & Co. AG
Franklinturm
Hofwiesenstrasse 349
8050 Zürich (CH)

(54) **SYSTEMS AND PROCESSES FOR SEPARATION OF COMPLEX FEEDS**

(57)    The present invention relates to field of separation processes, particularly to processes for separation of light hydrocarbons utilizing microporous molecular sieving membranes. The inventive process relies on a membrane system comprising at least two membrane units in sequential order, each membrane unit comprising a membrane where molecular sieves are tightly embedded in channels perpendicular to the membrane surface and the membrane comprises a polymer matrix and allows separating a target compound from a mixture of at least 3 compounds. The invention further provides for new membrane systems.

**EP 4 624 024 A1**

(52) Cooperative Patent Classification (CPC): (Cont.)

C-Sets
**C07C 7/005, C07C 11/06;**
**C07C 7/144, C07C 9/08;**
**C07C 7/144, C07C 11/06**

**Description**

The present invention relates to field of separation processes, particularly to processes for separation of light hydrocarbons from complex feed streams utilizing microporous molecular sieving membranes. The invention provides for new membrane systems, capable in separating such compound of interest from a complex feed stream. Further provided is an inventive process which relies on a membrane system comprising at least two membrane units in sequential order, each membrane unit comprising a membrane where different molecular sieves are tightly embedded in channels perpendicular to the membrane surface and the membrane comprises a polymer matrix.

Many chemical manufacture and refining processes involve the separation of a desired product compound from a fluid mixture containing two or more other compounds having physical properties in close proximity to those of the product compound. Examples of such separations include separation of propylene from other components, such as other alkanes, alkenes, hydrogen, CO2, nitrogen.

On commercial scale, these separations are performed by distillation. Distillation of such compounds, however, requires tall distillation towers and extensive heating to make a satisfactory separation, due to the close proximity of boiling points of the compounds being separated. Distillation separation consumes a significant quantity of energy. For example, separation of ethylene and propylene alone, the two largest volume chemicals produced worldwide, from ethane and propane by distillation has been estimated to consume 0.3% of global energy use.

On a commercial scale, mixed streams containing valuable product compounds, e.g. propylene, are often burned as conventional separation technology is not profitable.

On a development scale, separation of fluids by membranes is described. Stark et al (WO2017/207424) describes membranes comprising MOFs embedded in channel-like structures of a polymer matrix. These membranes find many applications, including separation of binary gas mixtures. Liu et al (US2019/0381449) describe a multistage membrane system and process for treating a binary gas stream, specifically CO2/CH4. In embodiments, the multistage membrane system comprises a combination of a microporous zeolitic inorganic membrane and a polymeric membrane. Lai et al (US2012/0310018) describe zeolitic imidazolate framework (ZIF) membranes useful in separating binary mixtures, such as $C_2$-hydrocarbons from $C_{3+}$ hydrocarbons. Pinnau et al (US6361582) describe the gas separation using $C_{3+}$ hydrocarbon resistant membranes. None of these separation methods advanced from development scale to commercial scale. The documents cited above disclose separation methods for binary mixtures.

However, none of the separation methods disclosed therein allow separation of a compound of interest from a complex feed, i.e. a feed comprising in addition to the compound of interest at least two other compounds.

There is an ever-existing need for providing alternative separation methods to those already existing in the field. Further, there is a significant need for improved separation techniques to address one or more drawbacks recognized in the field, specifically separating one product of interest (target molecule) from a complex feed stream comprising that target molecule. There is a further need for more modular and flexible separation processes. There is a particular need for separation processes being more energy efficient when compared to conventional distillation.

Thus, it is an object of the present invention to mitigate at least some of these drawbacks of the state of the art. In particular, it is an aim of the present invention to provide an improved method for separating hydrocarbons. It is a further aim to provide improved membrane systems, adapted to such methods.

These objectives are achieved by the process as defined in claim 1 and by the membranes as defined in claim 9. Further aspects of the invention are disclosed in the specification and independent claims, preferred embodiments are disclosed in the specification and the dependent claims. Accordingly, the invention relates

- in a first aspect to membrane systems (10);
- in a second aspect to a method for separating a gaseous hydrocarbon (specifically propene) from a mixture of at least three gaseous hydrocarbons (specifically a mixture comprising paraffins and olefins) by use of a membrane system (10);
- in a third aspect to the use of membrane systems in complex petrochemical applications and separation methods.

The present invention will be described in more detail below. It is understood that the various embodiments, preferences and ranges as provided / disclosed in this specification may be combined at will. Further, depending of the specific embodiment, selected definitions, embodiments or ranges may not apply.

Unless otherwise stated, the following **definitions** shall apply in this specification:

As used herein, the term "a", "an", "the" and similar terms used in the context of the present invention (especially in the context of the claims) are to be construed to cover both the singular and plural unless otherwise indicated herein or clearly contradicted by the context. As used herein, the terms "including", "containing" and "comprising" are used herein in their open, non-limiting sense. Percentages given in this specification, unless otherwise indicated, relate to mass - % (wt.%).

**Membrane:** The term membrane is known in the field and denotes a device that functions as a selective barrier due to the presence of pores / channels. Membranes may be present in the form of flat sheets or in the form of hollow fibers.

In each case, such membrane is defined by two surfaces and channels / pores perpendicular to the membrane surfaces.

Flat sheets are typically manufactured continuously and typically have a width of 0. 25 - 1.5 m and a thickness of 10 - 200 micrometers. Hollow fibres are typically manufactured continuously and have an outer diameter of 0.1 - 5 mm and typically have a wall thickness of 10 - 500 micrometers.

In the context of this invention, the polymer pore size / channel size of the membrane may cover a broad range, typically from 20 to 3000 nm, preferably from 100 to 500 nm.

A membrane is "self-supporting", if the membrane itself is free-standing and / or can be handled manually without a supporting structure. Further, a membrane may be part of a layered structure, i.e. comprise additional layers, such as supporting layers or covering layers. Such additional layers may improve stability of the membrane, allowing reliable manufacturing and / or use at higher pressures. MOFs and/or zeolites (both as outlined in further detail below) are embedded in these pores / channels, particularly channels vertical to the membrane plane. Without being bound to theory, it is believed that the MOFs substantially improve the membrane's selectivity.

The part of the feed passing the membrane is termed "permeate" while the part of the feed not passing the membrane is termed "retentate".

The term "ideal selectivity" is defined as the ratio of the permeance of the smaller compound (higher permeance) and the permeance of the larger compound (lower permeance) through a membrane and is a measure of the ability of a membrane to separate the two compounds. (For example, see Baker, Richard W., "Membrane Technology and Applications", pp. 290-291, McGraw-Hill, New York, 2000).

The selectivity of a membrane for the components of a given fluid may be assessed also by considering Knudsen-diffusion. Assuming a simple, non-size-selective pore / channel, diffusion is dependent on pore radius and molecular mass. The separation of compounds stemming from Knudsen diffusion can be calculated with the following formula, where alpha is the selectivity of compound A over compound B and Ma and Mb is the respective molar mass.

$$\alpha_{M_A/M_B} = \sqrt{\frac{M_B}{M_A}}$$

For example, the pair propylene / propane has a value of 1.023 and the pair ethylene / ethane has a value of 1.035. The inventive membranes show values way above the Knudsen limit, particularly a selectivity for the pair propylene / propane of at least 10 and the pair ethylene / ethane of at least 5. This finding indicates that the MOFs incorporated in the membrane significantly influence the selectivity of the membranes. On the other hand, membranes showing a selectivity in the range of the Knudsen limit apparently possess simple pores / channels that only provide moderate selectivity, i.e. a selectivity not influenced by molecular sieving. To distinguish from simple membranes, having a selectivity in the range of the Knudsen limit, the expression "tightly embedded MOFs" is chosen. The term "pore size range" is known in the field and may be determined by measuring the permeance of reference gaseous molecules of various sizes. By consecutively measuring from very small compounds, i.e. H2, to larger compounds, i.e. propane, the range where the membrane is selective can be found. The effective pore size range of a membrane is then determined as being between the size of two consecutive compounds having a permeance difference of at least factor 5.

**Metal-organic frameworks (MOFs):** The term MOFs is known in the field and denotes chemical entity comprising metal ions (including clusters) cores and ligands (also termed "struts"). The ligands have at least two coordination sites. In the context of this invention MOFs do form a 3-dimensional network with open pores.

**Zeolites:** The term zeolites is known in the field and particularly denotes microporous aluminosilicate minerals, both natural and synthetic. Compared to MOFs, they are free of organic ligands.

**Hydrocarbons:** The term hydrocarbon is known in the field and denotes molecules consisting of hydrogen and carbon. Accordingly, the term hydrocarbon includes alkanes, alkenes (including multiple double bonds such as dienes) and alkines. Further, aliphatic and cyclic molecules are included. By $C_n$ hydrocarbons, reference is made to hydrocarbons consisting of n carbon atoms. Accordingly, by $C_{n+}$ hydrocarbons, reference is made to hydrocarbons consisting of at least n carbon atoms. As conventional in the field, the term hydrocarbons shall not include aromatic molecules.

The present invention will be better understood by reference to the **figures.**

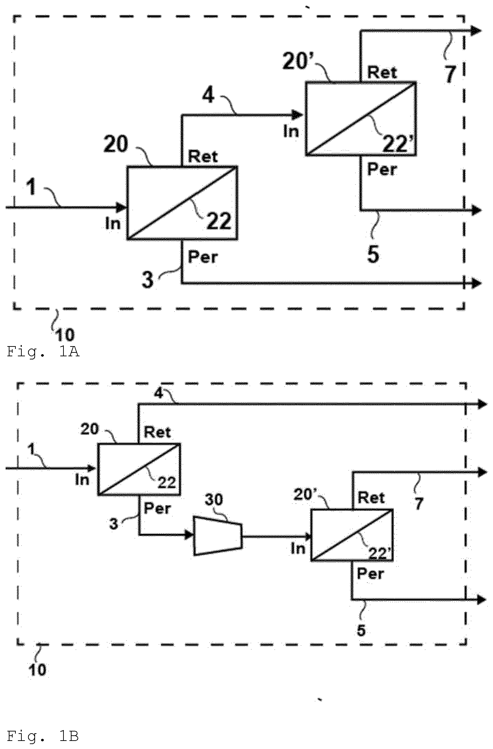

Figure 1 outlines the inventive separation process described herein.
List of references:

| (10) | membrane system with sensors (11, not shown), and control unit (12, not shown) |
|---|---|
| (20) and (20') | membrane separation units, sequentially aligned, with membrane modules (21, 21') comprising membrane(s) (22, 22') (the modules not shown) |
| (1) | feed |

(3)          permeate I,
(4)          retentate I
(5)          permeate II,
(7)          retentate II,
(21)        modules,
(30)        compressor,
(In)         feed inlet,
(Per)       outlet for permeate,
(Ret)       outlet for retentate.

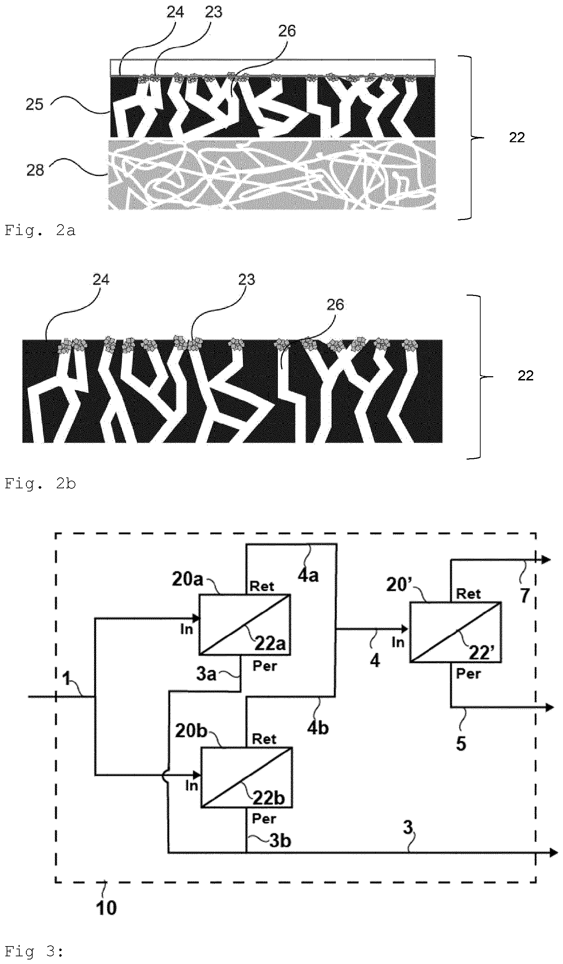

**Figure 2** schematically outlies embodiments of suitable membranes (22), where
Fig. 2a shows an embodiment with backing layer (28);
Fig. 2b shows an embodiment without backing layer.
List of references:

(22)        membrane
(23)        molecular sieves;
(24)        polymer matrix;
(25)        1st functional layer [comprising polymer matrix (24) with channels (26) and molecular sieves (23) tightly embedded in said channels (26)];
(26)        channels perpendicular to the membrane surface.
(28)        backing layer connected to said 1st functional layer (25), [improving pressure and tear resistance].

**Figure 3** outlines a further embodiment of the inventive separation process described herein, where a single module 22 is replaced by a combination of modules 22a and 22b in parallel mode.

In a **first aspect,** the invention relates to membrane systems (10) and production plants, comprising membrane systems as described herein. These membrane systems allow separation of complex gas mixtures, i.e. a gas comprising of at least 3 components where typically one component is of interest. This aspect of the invention shall be explained in further detail below:

**Membrane systems (10):**

In line with the present invention, the membrane system (10) comprises at least two membrane units (20, 20') in sequential order. Membrane systems may include known membranes, membrane modules and membrane units. As shown in fig. 1, a feed is subjected to a first membrane unit (20), thereby obtaining a retentate I and a permeate I. Either retentate I (shown in fig. 1A) or permeate I (shown in fig. 1B) are subjected to a second membrane unit (20') thereby obtaining retentate II and permeate II. Depending on the selection of membranes and starting material used, either retentate II or permeate II is the desired product. The combination of two membrane units allows for both, high flexibility and high selectivity. Membranes according to the invention are described in detail below. The careful selection of the molecular sieve's pore size PS is considered an important feature of the membrane system to allow a reliable separation of complex gas streams as defined below. Suitable pore size for molecular sieves (23, 23') of membrane 22 and 22' are as follows:

$2.9 \leq PS_{23} \leq 4.46$ Angström and
$2.9 \leq PS_{23'} \leq 4.50$ Angström, and
the pore size difference $\Delta PS$ between said molecular sieves being between 0.1 to 1.5 Angström. Depending on the specific separation task, molecular sieves (23, 23') may be selected to comply with a $\Delta PS$ preferably in the range 0.1 to 0.3 Angström, more preferably in the range of 0.1 to 0.15 Angström. It was found that the membranes used in the inventive system allow adjusting to such specific pore size.

Thus, the combination of the two types of membranes in the specified arrangement in the present invention allows separation of a compound of interest (ie. a target compound such as propene) from a complex feed (such as a gas stream comprising propene and at least two other gaseous components) .

Further, it is possible to include a multitude of first membrane units (20) in parallel into the membrane system (10). Conversely, it is also envisaged to include a multitude of 2nd membrane units (20') in parallel into the membrane system (10). Such embodiments may account for different feed volumes (1, 4, according to fig. 1A or 1, 3 according to fig. 1B) or permeances in the individual membranes (22, 22'). Such embodiment is shown in fig. 3, where membrane unit (20) is replaced by two membrane units (20a, 20b).

In line with the present invention, the membrane system (10) comprises at least two membrane units (20, 20') in sequential order. Membrane systems are explained in further detail below. Further, it is possible to include in the membrane system one or more **sensors** (11, 11') adapted to measure and transmit a property of feed (1), retentate (4, 7) and / or permeate (3, 5) and a control unit (12) adapted to receive such signals. Such property may be the concentration of one or more components in the respective stream, the mass flow of said stream, the temperature of said stream and/or the pressure of said stream. This embodiment allows to predict performance and maintenance of the process, e.g. by interpreting differences of the properties measured over time, or by comparing differences of the properties with pre-defined values.

Further, it is possible to include in the membrane system one or more **compressors** (30), preferably located between 20 and 20'. Presence of a compressor may increase performance of the membrane system and/ or facilitate integration of the inventive membrane system into a larger plant.

In embodiments, the invention pertains to a membrane system (10) as described herein, wherein the pore size of the molecular sieves (23, 23') increases from first to second unit ($PS_{23'} > PS_{23}$), and the inlet of the second membrane unit ($In_{20'}$) is in fluid communication with second outlet for retentate of the first membrane unit ($Ret_{20}$). This embodiment is depicted in Fig 1A.

In embodiments, the invention pertains to a membrane system (10) as described herein, wherein the pore size of the molecular sieves (23, 23') decreases from first to second membrane unit ($PS_{23'} < PS_{23}$), and the inlet of the second membrane unit ($In_{20'}$) is in fluid communication with the first outlet for permeate of the first membrane unit ($Per_{20}$). This embodiment is depicted in Fig. 1B.

Particularly preferred membrane system (10) are described in the following embodiments, wherein in each case the ideal selectivity and the pore size are determined measuring the flow of the specified gas in a test cell; and wherein in each case the membranes are available from Unisieve AG under the trade name given below:

Embodiment 1, where:

- the first membrane unit (20) comprises a membrane (22) having pore size of 2.9 - 3.9 Angstrom **(UniS-3.9)** with an ideal selectivity of at least 10, preferably at least 20 when using ethylene and propylene as reference gas; and
- the second membrane unit (20') comprises a membrane (22') having pore size of 4.1 - 4.5 Angstrom **(UniS-4)** with an ideal selectivity of at least 10, preferably at least 20, when using propylene and propane as reference gas.

This embodiment is particularly suited to separate propene as a target compound from other gases, specifically to separate propene from propane and ethene.

Embodiment 2, where:

- the first membrane unit (20) comprises a membrane (22) having pore size of 2.9 - 3.5 Angstrom **(UniS-3)** with an ideal selectivity of at least 20, preferably at least 30 when using N2 and propylene as reference gas; and
- the second membrane unit (20') comprises a membrane (22') having pore size of 4.1 - 4.5 Angstrom **(UniS-4)** with an ideal selectivity of at least 10, preferably at least 20, when using propylene and propane as reference gas).

This embodiment is particularly suited to separate propene as a target compound from other gases, specifically to separate propene from N2 and propane.

Embodiment 3, where:

- the first membrane unit (20) comprises a membrane (22) having pore size of 2.9 - 3.2 Angstrom (UniS-2) with an ideal selectivity of at least 20, preferably at least 30, when using H2 and propylene as reference gas; and
- the second membrane unit (20') comprises a membrane (22') having pore size of 4.1 - 4.5 Angstrom (UniS-4) with an ideal selectivity of at least 10, preferably at least 20, when using propylene and propane as reference gas.

This embodiment is particularly suited to separate propene as a target compound from other gases, specifically to separate propene from hydrogen and propane.

**Membrane (22, 22'):**

Without being bound to theory, it is believed that the membranes (22, 22') described herein play an important role in successfully performing the continuous process described herein. Particularly, the combination of polymer matrix (24), channels (26) and molecular sieves (23) are important. The molecular sieves (23) remain within said channels (26) when used, ie. during manufacturing of membrane modules and when in use. To distinguish from simple (and removable) coatings of molecular sieves on membrane surfaces, the expression "tightly embedded" is used.

As outlined below, membranes (22, 22') are assembled in membrane modules (21, 21'), which are the key elements of membrane units (20, 20'). In line with the present invention, at least two membrane units are combined to a membrane system (10). The use of such membrane system (10) allows for improved separation of specific products from a feed on large scale, avoiding the need for distillation and ensuring a reliable separation over a long term, along with a high degree of flexibility.

The membranes (22) may be present in the form of flat sheets or in the form of fibers. According to this invention, membrane (22) comprises a first functional layer (25), optionally a backing layer (28) connected to the first functional layer. The membrane (22) may further comprise a 2nd functional layer (27) opposite to said backing layer (28).

First functional layer (25): This layer comprises a polymer matrix (24) and molecular sieves (23) and said matrix (24) comprising channels perpendicular to the membrane surface thereby connecting both surfaces of said membrane and wherein said molecular sieves (23) are located within said channels. Due to the location of molecular sieves (23) within said channels, the molecular sieves are tightly embedded and the membranes show a selectivity for a given product / feed pair above the Knudsen limit.

Matrix: A suitable polymer matrix (24) may be selected from a broad range of polymers, including celluloseacetate (CA), polyethersulfone (PES), polyvinylidenfluoride (PVDF), polysulfone (PSf), polyacrylonitrile (PAN), polyimide (PI), polyamide-imide (PAI, e.g., TORLON), polyether-etherketones (PEEK) and polylactic acid (PLA), polycarbonates (PC), polystyrenes (PS), polyacrylates, and polyurethanes (PU). A preferred polymer for matrix (24) is PES.

Molecular Sieves: The Molecular sieve (23) may be selected from a broad range of known molecular sieves, including molecular frameworks (MOFs) and optionally zeolites. Suitable MOFs include ZIF-7, ZIF-8, ZIF-L, ZIF-20, ZIF-21, ZIF-22, ZIF-23, ZIF-67, ZIF-69, ZIF-71, ZIF-90, SIM-1, MIL-47, MIL-53, MOF-5, MIL-96, MIL-89, MIL-101, and HKUST-1 and combinations thereof; preferably ZIF-8, ZIF-90 and ZIF-7 and combinations thereof. Suitable zeolites include AlPO-14, AlPO-18, AlPO-34, SAPO-34, SSZ-13, DD3R, ITQ-12, CDS-1, UZM-25 and combinations thereof. Molecular sieves are selected to provide a suitable size exclusion for performing the separation task. As a consequence, membrane (22) and (22') differ; preferably in the choice of molecular sieve, optionally also in pore size, matrix, backing layer or other parameters.

Backing layer: A broad range of materials may be used. It is understood that the backing layer is porous, not negatively influencing selectivity of the functional layer (25). The backing layer (28) may comprise or consist of a polyamide membrane. Advantageously, the membrane (22) comprises or consists of a first functional layer (25) and a backing layer (28).

Second functional layer: A second functional layer may further improve performance of the membrane. If present, the second functional layer (27) may comprise or consist of molecular sieves as defined herein.

Membrane morphology: Advantageously, the channel-like structures (26) have a diameter of 50 - 10 000 nm, preferably 100 - 1000 nm. Advantageously, the channel-like structures (26) account to 0.5 - 50 vol-% of the membrane (22, 22'), preferably account to 20 - 50 vol% of the membrane.

In a preferred embodiment, the membrane (22, 22') comprises backing layer (28), preferably selected from polyamide membranes with pore size above 3 micrometers; a first functional layer (25) comprising a polymer matrix (24) and molecular sieves (23), said functional layer (25) comprising channels (26) within said matrix (24) perpendicular to the membrane surface thereby connecting both surfaces of said membrane and wherein said molecular sieves (23) are located within said channels (26), said molecular sieves (23) are as defined above, and said polymer matrix (24) is as defined above. Advantageously, the backing layer (28), is selected from polyamide membranes with pore size above 3 micrometers. Advantageously, the matrix (24) is a PES matrix. Advantageously, the molecular sieve (23) is ZIF-8.

**Membrane module (21, 21'):**

Typically, one or more membranes (22, 22') are housed in a membrane module (21, 21') as known in the field. Such membrane modules include Spiral-wound membrane modules, plate-frame modules, cylinder modules and potted hollow-fiber modules. Spiral-wound membrane modules comprising one membrane are preferred. The making of all these types of modules is well known in the art. Since conventional polymeric materials are used for the membranes, they are relatively simple and inexpensive to prepare and to house in modules, particularly compared with other types of membranes, such as pyrolyzed carbon membranes or ceramic membranes.

**Membrane unit (20, 20'):**

Membrane units are known per se; a membrane unit (20) comprises one or more membrane modules (21, 21'). Each membrane module, in turn, comprises one or more membranes (22, 22'). Membrane units further comprise at least one inlet (In), one outlet for retentate (Ret) and one outlet for permeate (Per).

Membrane units (20) are adapted to elevated temperatures, e.g. 30 - 100°C and elevated pressures, e.g. 10 - 50 bar.

In a preferred embodiment, the membrane unit (20, 20') comprises one or more modules (21, 21') as described above, along with feed inlet, permeate outlet, retentate outlet and one or more sensing elements (11) as described herein.

In more general terms, in a **second aspect,** the invention relates to a process for separating a feed stream (1), into three or more product streams. By performing this process, one of said product streams is obtained where the concentration of a target compound is increased when compared to the concentration of that compound in the feed stream (1). This aspect of the invention shall be explained in more detail below.

**General:**

The process is a continuous process, as indicated by the terms feed stream and product stream(s). The process may be implemented in existing manufacturing processes, The process is very versatile and may be implemented to separate a wide range of products from corresponding starting materials (feed stream (1)).

Separation via membranes is a known technology. By sending said feed stream (1) through a membrane (22) of membrane system (10), a feed is separated into a permeate stream and a retentate stream. This step may be performed in a wide range of temperatures and pressures. Suitable parameters may be identified by the skilled person considering the separation task, the type of membrane and of membrane system. Suitable temperatures are in the range of 0-120°C, suitable pressures are in the range of 0-30 bar.

The hydrocarbon stream obtained may be subjected to further process steps, such as compression. Such steps are known in the field.

In embodiments, the process is adapted to separate a feed stream (1), into three or more product streams, wherein said feed stream comprises a mixture containing three or more gases selected from the group consisting of hydrogen, nitrogen, methane, C2 hydrocarbons, C3 hydrocarbons and C4 hydrocarbons; and said process comprises the step of sending said feed stream (1) through a membrane system (10) as defined herein, 1st aspect of the invention to thereby obtain 3 product streams, one of which contains the target compound in an increased concentration when compared to feed stream (1).

The inventive process, which makes use of the tunable pore size of molecular sieving membranes as outlined herein, enables the separation of higher value hydrocarbons from a complex hydrocarbon mixture via a size-exclusion mechanism. Compared to prior art, this membrane assisted separation process can purify a desired hydrocarbon compound from complex mixtures solely by membrane technology.

In embodiments, the invention thus provides for a separation process as described herein not containing distillation steps.

In embodiments, the invention thus provides for a separation process as described herein not containing extraction steps. Thus, the inventive process allows for the separation of unsaturated organic compounds from fluid mixtures without requiring the presence of water during the separation.

The process also differs from previous processes in that the separation is purely dependent on the molecular diameter of the to be separated compounds, separating the mixture according to the chosen molecular sieve pore size.

**Membrane Systems and Membranes:**

Suitable membrane systems and membranes are specified above, first aspect of the invention. It was found advantageous if said membrane (22, 22') comprises or consists of a backing layer (28); and a functional layer (25), said functional layer (25) comprising said polymer matrix (24) and said molecular sieves (23) located in said channels (26).

In embodiments, said polymer matrix (24) comprises or consists of polysulfones, polyethersulfones, sulfonated polysulfones, sulfonated polyether sulfones, polyetherimides, cellulosic polymers, polyimides, polyamide/imides, polyketones, polyether ketones, poly(arylene oxides), poly(benzobenzimidazole), polyhydrazides, polyoxadiazoles, polybenzoxazoles, polytriazoles, poly(benzimidazole), polycarbodi-imides, polyphosphazines, microporous polymers, and mixtures thereof. Advantageously, the polymer matrix consists of PES polymer.

In embodiments, said molecular sieves (23, 23') are selected from metal organic frameworks (MOFs) and optionally zeolites; where said MOFs preferably selected from the group consisting of ZIF-7, ZIF-8, ZIF-L, ZIF-20, ZIF-21, ZIF-22, ZIF-23, ZIF-67, ZIF-69, ZIF-71, ZIF-90, SIM-1, MIL-47, MIL-53, MOF-5, MIL-96, MIL-89, MIL-101, and HKUST-1 and mixtures thereof; and said zeolites preferably selected from the group consisting of AlPO-14, AlPO-18, AlPO-34, SAPO-34, SSZ-13, DD3R, ITQ-12, CDS-1, UZM-25 and mixtures thereof.

**Feed stream (1):**

A wide range of starting materials (feed stream), comprising hydrocarbons as defined herein, may be subject to the inventive process. Such starting materials include interim products of oil refineries or other chemical production plants. They may contain hydrocarbons as defined herein and further components, such as CO, CO2, H2, N2 and noble gases.

In an embodiment, the feed stream is selected from the feed, head stream, or bottom stream of a hydrocarbon fractionator, particularly a C3 fractionator. In an embodiment, the feed stream is a flue gas. In an embodiment, the feed stream is a flare stream. In an embodiment, the feed stream is a purge gas stream, such as purge gas from PP formed. In an embodiment, the feed stream is a recycled stream of a polypropylene reactor. It is considered beneficial that the inventive process tolerates a large variety of starting materials and combination of various streams occurring on a commercial production site is possible.

**Product stream / Target compound:**

The product stream contains the target compound(s), e.g. C2 or C3 or C4, hydrocarbons in a concentration above the concentration of the corresponding compound in the feed (1). Thus, the target compound is up-concentrated compared to the feed. The purity of the target compound depends on the intended use, typically above 60 wt%, such as above 90 wt%, above 95 wt%, above 99 wt%, above 99.5 wt% above 99.9 wt%. In embodiments, the concentration is chemical grade of the respective compound. In embodiments, the concentration is polymer grade of the respective compound.

The term C2 product includes Acetylene, Ethylene, Ethane. The term C3 product includes Methylacethylene, Propadiene, Propene and Propane. The term C4 product includes n-Butane, iso-Butane, 1-Butene, iso-Butene, Butadiene, 2-cis-Butene and 2-trans-Butene. A particularly preferred product is propene.

**Propene Separation:**

Specifically, the inventive process allows for the separation of propene as the target compound. In such embodiment, the feed (1) comprises propene, and one of said product streams contains propene in a higher concentration than feed (1). Preferably, the product stream comprises propene in refinery grade (RGP, ~60-70%), in chemical grade (CGP, 92-96%) or in polymer grade (PGP, >99.5%).

In an embodiment, said feed stream (1) contains (i) 1 - 99 wt % propene and (ii) 99 - 1 wt% propane and (iii) 99 - 1 wt% other gases selected from nitrogen, hydrogen and methane; and (i) + (ii) + (iii) sum up to 100%.

In a further embodiment, said feed stream (1) contains 1 - 99 wt % propene and 99 - 1 wt% other hydrocarbons, said other hydrocarbons preferably selected from acetylene, ethylene, ethane, methylacethylene, propadiene, propane, n-butane, iso-butane, 1-butene, iso-butene, butadiene, 2-cis-Butene, and 2-trans-butene.

The invention particularly provides for a process as described herein for separation of the following gas pairs:

- C3H6 / C3H8 / ethene;
- C3H6 / C3H8 / nitrogen;
- C3H6 / C3H8 / hydrogen;
- C3H6 / C3H8 / methane;
- C3H6 / C3H8 / methane + hydrogen + nitrogen + ethene.

Depending on the feed and the membrane used, the inventive process may be implanted in different ways for separating propene. In a first embodiment, propene is retentate I, while in the second embodiment, propene is permeate I.

Thus, in a **first embodiment,** the invention relates to a process where a first membrane unit (20) comprises a membrane (22) that separates propene and $C_{3+}$ hydrocarbons (4, ret I) from methane, ethane, ethene, ethine, hydrogen, CO, $CO_2$ (3, per I) and a second membrane unit (20') comprises a membrane (22') that separates propene (5, per II) from propane and $C_{4+}$ hydro-carbons(7, ret II). Advantageously, membranes (22) have an ideal selectivity of at least 10, preferably at least 20. Advantageously, membranes (22') have an ideal selectivity of at least 10, preferably at least 15. (Fig. 1A)

Thus, in a **second embodiment,** the invention relates to a process where a first membrane unit (20) comprises a membrane (22) that separates methane, ethane, ethene, ethine, hydrogen, CO, CO2 and propene (permeate I) from propane and C4+ hydrocarbons (retentate I); and a second membrane unit (20') comprises a membrane (22') that separates propene (7, retentate II) from methane, ethane, ethene, ethine, H2, CO, and CO2 (per II). Advantageously, membranes (22) have an ideal selectivity of at least 10, preferably at least 15. Advantageously, membranes (22') have an ideal selectivity of at least 10, preferably at least 20. (Fig 1B)

In a **third aspect,** the invention relates to the use of membrane systems (10), as described herein. This aspect of the invention shall be explained in further detail below:

The systems as described herein are useful for separation of a C2 hydrocarbon, a C3 hydrocarbon or a C4 hydrocarbon from a feed stream comprising such hydrocarbon along with a multitude of other components / impurities, such as 3-component systems, 4-component systems and so forth. The desired product may be obtained avoiding distillation and avoiding the use of water. Further, the desired product may be obtained in high purity, as defined above, 2nd aspect of the invention.

Accordingly, the invention also provides for the use membrane systems (10), each as described herein, for C3H6 separation, i.e. removing two or more by-products / impurities from the desired product C3H6. This separation is particularly useful in the context of propylene polymerisation. Typical propene streams show a purity as discussed above, 2nd aspect. Typical feed streams are

- C3H6 / C3 / ethene;
- C3H6 / C3 / nitrogen;
- C3H6 / C3 / hydrogen;
- C3H6 / C3 / methane; and
- C3H6 / C38 / methane + hydrogen + nitrogen + ethene.

To further illustrate the invention, the following, non-limiting **example** is provided.

**Example A,** Separation of Propylene from Propane and Ethylene. The goal is to purify the propylene out of a feed stream, i.e. separate it from smaller and a larger compounds, both being very close in size.

In this example, two different molecular sieving membrane units are connected, resulting in the membrane system described above and shown in figure 1A. In this example, the first membrane unit (22) comprises one spiral wound module and the membrane has a surface of 0.3 m2. The module used is '1812-UniS-3.9' and can be obtained from UniSieve AG. In this example, the second membrane unit (22') comprises a spiral wound module and the membrane has a surface of 0.05 m2. The module used is '1812-UniS-4' and can be obtained from UniSieve AG. The feed is a hydrocarbon gas mix containing ethylene, propylene and propane. Ethylene has a kinetic diameter of 3.9 Angström, propylene of 4.0 Angström and propane of 4.2 Angström. In detail, a first membrane unit with a pore size of 3.9 Angström is separating ethylene (permeate I) from propylene and propane (retentate I). A second membrane unit is coupled to the stream 4, with a pore size of 4.1 Angström, effectively separating propylene (permeate II) from propane (retentate II). In this example, the feed pressure used is 7 barg, the permeate pressure is at 0 barg and the temperature is ambient. The mass flows are controlled and measured with Coriolis type mass flow controllers. The gas compositions of the various streams are determined with gas chromatography using a flame ionization detector, the results being summarized in the table below. The stream numbers correspond to Figure 1A.

|  | 1 Feed | 3 Ethylene enriched | 5 Propylene enriched | 7 Propane enriched |
|---|---|---|---|---|
| Pressure (barg) | 7 | 0 | **0** | 6.85 |
| Temperature (°C) | 24 | 24 | **24** | 24 |
| Mass flow (g/h) | 11 | 6.0 | **3.2** | 1.8 |
| Ethylene (mol%) | 15.0 | 26.4 | **0.3** | 0.0 |
| Propylene (mol%) | 80.0 | 71.9 | **98.8** | 74.9 |
| Propane (mol%) | 5.0 | 1.6 | **0.8** | 25.1 |

In conclusion, propylene can be effectively separated via this membrane system from both, components that have a smaller kinetic diameter and components that have a larger kinetic diameter.

**Claims**

1. A membrane system (10) comprising a first membrane unit (20), a second membrane unit (20') in fluid connection;

said membrane units (20, 20') are arranged in sequential order and the first membrane is arranged first in line towards a feed stream (1);
each membrane unit (20, 20') comprises membranes (22, 22') at least one inlet (In), at least a first outlet for permeate (Per) and at least a second outlet for retentate (Ret), wherein

• said membrane (22, 22') comprises a polymer matrix (24) and molecular sieves (23, 23'), and
• said membrane (22, 22') comprises channels (26) within said matrix (24) perpendicular to the membrane surface thereby connecting both surfaces of said membrane, and
• said molecular sieves (23, 23') are tightly embedded within said channels (26), and
• said molecular sieves (23, 23')

  o each having a defined pore size $PS_{23}$, $PS_{23'}$ where $2.9 \leq PS_{23} \leq 4.50$ Angström and $2.9 \leq PS_{23'} \leq 4.50$ Angström, and
  o the pore size difference $\Delta PS$ between said molecular sieves being between 0.1 to 1.5 Angström.

2. The membrane system (10) as defined in claim 1, the system further comprising:

• one or more sensors (11, 11') adapted to measure and transmit a property of feed (1), retentate (4, 7) and/or permeate (3, 5) and a control unit (12) adapted to receive such signals and to predict performance and maintenance of the process by interpreting differences of the properties measured over time; and / or
• a compressor (30) located between first and second membrane unit.

3. A membrane system (10) according to any of claims 1 to 2, where each membrane separation unit (20, 20') comprises one or more modules (21, 21'), wherein said module is selected from

• spiral wound membrane modules,
• plate frame membrane modules,
• cylinder modules, and
• potted hollow membrane fibre membrane modules, in each case comprising one or more membranes (22, 22') as defined in claim 1.

4. A membrane system (10) according to any of claims 1 to 3, wherein

• two or more separation units (20a, 20b) are arranged in parallel and /or
• two or more separation units (20'a, 20'b) are arranged in parallel.

5. A membrane system (10) according to any of claims 1 to 4, wherein

• the pore size of the molecular sieves (23, 23') increases from first to second unit ($PS_{23'} > PS_{23}$), and
• the inlet of the second membrane unit ($In_{20'}$) is in fluid communication with second outlet for retentate of the first membrane unit ($Ret_{20}$).

6. A membrane system (10) according to any of claims 1 to 4, wherein

• the pore size of the molecular sieves (23, 23') decreases from first to second membrane unit ($PS_{23'} < PS_{23}$), and
• the inlet of the second membrane unit ($In_{20'}$) is in fluid communication with the first outlet for permeate of the first membrane unit ($Per_{20}$).

7. A membrane system (10) according to any of claims 1 to 6, wherein

in a first embodiment:

• the first membrane unit (20) comprises a membrane (22) having pore size of 2.9 - 3.9 Angstrom with an ideal selectivity of at least 10, preferably at least 20 (using ethylene and propylene as reference gas); and
• the second membrane unit (20') comprises a membrane (22') having pore size of 4.1 - 4.5 Angstrom with an ideal selectivity of at least 10 (using propylene and propane as reference gas);

in a second embodiment:

• the first membrane unit (20) comprises a membrane (22) having pore size of 2.9 - 3.5 Angstrom with an ideal selectivity of at least 20, preferably at least 30 ($N_2$ and propylene as reference gas); and
• the second membrane unit (20') comprises a membrane (22') having pore size of 4.1 - 4.5 Angstrom with an ideal selectivity of at least 10 (using propylene and propane as reference gas); or

in a third embodiment:

• the first membrane unit (20) comprises a membrane (22) having pore size of 2.9 - 3.2 Angstrom with an ideal selectivity of at least 20, preferably at least 30 (H2 and propylene as reference gas); and
• the second membrane unit (20') comprises a membrane (22') having pore size of 4.1 - 4.5 Angstrom with an ideal selectivity of at least 10 (using propylene and propane as reference gas); and

wherein in each case the ideal selectivity and the pore size are determined measuring the flow of the specified gas in a test cell.

8. A process for separating a feed stream (1), into three or more product streams, wherein

said feed stream comprises a mixture containing three or more gases selected from the group consisting of hydrogen, nitrogen, methane, $C_2$ hydrocarbons, $C_3$ hydrocarbons and $C_4$ hydrocarbons;
said process comprises the step of sending said feed stream (1) through a membrane system (10) as defined in any of claims 1 - 8 to thereby obtain three product streams.

9. The process according to claim 8, wherein

• the feed (1) comprises propene, and
• one of said products streams contains propene in a higher concentration than feed (1), preferably propene in refinery grade, or chemical grade or polymer grade.

10. The process according to claim 9, wherein
said feed stream (1) contains (i) 1 - 99 wt % propene and (ii) 99 - 1 wt% propane and (iii) 99 - 1 wt% other gases selected from nitrogen, hydrogen and methane; and (i) + (ii) + (iii) sum up to 100%.

11. The process according to any of claims 8 to 10, wherein said feed stream (1) is selected from

• the feed, head stream, or bottom stream of a hydrocarbon fractionator, particularly a C3 fractionator;
• a flue gas; or
• a flare stream; or
• a purge gas stream, particularly a purge gas from PP formed; or
• a recycle stream of a polypropylene reactor.

12. The process according to any of claims 8 to 11, wherein said membrane (22, 22') comprises or consists of:

• a backing layer (28)
• a functional layer (25), said functional layer (25) comprising said polymer matrix (24) and said molecular sieves (23) located in said channels (26).

13. The process according to any of claims 1 to 5, where

said polymer matrix (24) comprises or consists of polysulfones, polyethersulfones, sulfonated polysulfones, sulfonated polyether sulfones, polyetherimides, cellulosic polymers, polyimides, polyamide/imides, polyketones, polyether ketones, poly(arylene oxides), poly(benzobenzimidazole), polyhydrazides, polyoxadiazoles, polybenzoxazoles, polytriazoles, poly(benzimidazole), polycarbodi-imides, polyphosphazines, microporous polymers, and mixtures thereof; and / or
said molecular sieves (23, 23') are selected from metal organic frameworks (MOFs) and optionally zeolites;

said MOFs preferably selected from the group consisting of ZIF-7, ZIF-8, ZIF-L, ZIF-20, ZIF-21, ZIF-22, ZIF-23, ZIF-67, ZIF-69, ZIF-71, ZIF-90, SIM-1, MIL-47, MIL-53, MOF-5, MIL-96, MIL-89, MIL-101, and HKUST-1 and mixtures thereof;
said zeolites preferably selected from the group consisting of AIPO-14, AIPO-18, AIPO-34, SAPO-34, SSZ-13, DD3R, ITQ-12, CDS-1, UZM-25 and mixtures thereof.

14. Use of a membrane system (10) as defined in any of claims 1 to 8 for performing a process selected from

▪ C3H6 / C3H8 / ethene separation;
▪ C3H6 / C3H8 / nitrogen separation;

- C3H6 / C3H8 / hydrogen separation;
- C3H6 / C3H8 / methane separation;
- C3H6 / C3H8 / methane + hydrogen + nitrogen + ethene separation

in each case resulting in a propene stream of at least 90 wt% purity.

Fig. 1A

Fig. 1B

Fig. 2a

Fig. 2b

Fig 3:

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/381449 A1 (LIU CHUNQING [US] ET AL) 19 December 2019 (2019-12-19) | 2,3,5,6, 8-11,13, 14 | INV. B01D53/22 B01D69/14 |
| Y | * figures 1-6 * <br> * claims 1,2,3,5 * <br> * paragraph [0023] * | 1 | C07C7/144 |
| Y | DE 11 2021 002379 T5 (NGK INSULATORS LTD [JP]) 26 January 2023 (2023-01-26) <br> * claims 3,7 * | 1 | |
| A | DIVYA NAGARAJU: "In situ growth of metal-organic frameworks on a porous ultrafiltration membrane for gas separation", <br> JOURNAL OF MATERIALS CHEMISTRY A, <br> vol. 1, no. 31, <br> 2 January 2013 (2013-01-02), page 8828, <br> XP093168187, <br> GB <br> ISSN: 2050-7488, DOI: 10.1039/c3ta10438a <br> * abstract * | 1 | |
| | | | **TECHNICAL FIELDS SEARCHED (IPC)** |
| X | US 2024/058769 A1 (SCHNEIDER ELIA [CH] ET AL) 22 February 2024 (2024-02-22) | 12 | B01D C07C D01D |
| A | * figure 2a * <br> * paragraph [[0048]] * | 1 | |
| A | TING WU: "Microporous Crystalline Membranes for Kr/Xe Separation: Comparison Between AlPO-18, SAPO-34, and ZIF-8", <br> ACS APPLIED NANO MATERIALS, <br> vol. 1, no. 1, <br> 18 December 2017 (2017-12-18), pages 463-470, XP0093167161, <br> ISSN: 2574-0970, DOI: 10.1021/acsanm.7b00343 <br> * page 467 - paragraph 2 * | 1 | |

-/--

The present search report has been drawn up for all claims

| Place of search | Date of completion of the search | Examiner |
|---|---|---|
| Munich | 13 June 2024 | Sandin Rodriguez, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

**Application Number**

EP 24 16 6925

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/244211 A1 (JAPAN OIL GAS & METALS JOGMEC [JP]) 26 December 2019 (2019-12-26) * figures 1-3 * ----- | 4 | |
| X | US 2011/009684 A1 (DIAZ ZAIDA [US] ET AL) 13 January 2011 (2011-01-13) * figure 2 * ----- | 7 | |

TECHNICAL FIELDS
SEARCHED     (IPC)

The present search report has been drawn up for all claims

| Place of search | Date of completion of the search | Examiner |
|---|---|---|
| Munich | 13 June 2024 | Sandin Rodriguez, R |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

................................................................

& : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 24 16 6925

This annex lists the patent family members relating to the patent documents cited in the above-mentioned European search report.
The members are as contained in the European Patent Office EDP file on
The European Patent Office is in no way liable for these particulars which are merely given for the purpose of information.

13-06-2024

| Patent document cited in search report | Publication date | Patent family member(s) | Publication date |
|---|---|---|---|
| US 2019381449 A1 | 19-12-2019 | EP 3806987 A1 | 21-04-2021 |
| | | MY 194661 A | 12-12-2022 |
| | | US 2019381449 A1 | 19-12-2019 |
| | | WO 2019241722 A1 | 19-12-2019 |
| DE 112021002379 T5 | 26-01-2023 | CN 115720530 A | 28-02-2023 |
| | | DE 112021002379 T5 | 26-01-2023 |
| | | JP 7444990 B2 | 06-03-2024 |
| | | JP WO2022014469 A1 | 20-01-2022 |
| | | US 2023108642 A1 | 06-04-2023 |
| | | WO 2022014469 A1 | 20-01-2022 |
| US 2024058769 A1 | 22-02-2024 | EP 4015069 A1 | 22-06-2022 |
| | | EP 4243969 A1 | 20-09-2023 |
| | | KR 20230121744 A | 21-08-2023 |
| | | US 2024058769 A1 | 22-02-2024 |
| | | WO 2022129063 A1 | 23-06-2022 |
| WO 2019244211 A1 | 26-12-2019 | JP 6940206 B2 | 22-09-2021 |
| | | JP WO2019244211 A1 | 25-03-2021 |
| | | MY 188660 A | 22-12-2021 |
| | | US 2021129074 A1 | 06-05-2021 |
| | | WO 2019244211 A1 | 26-12-2019 |
| US 2011009684 A1 | 13-01-2011 | AU 2009203713 A1 | 16-07-2009 |
| | | BR PI0907244 A2 | 14-07-2015 |
| | | CA 2711477 A1 | 16-07-2009 |
| | | CN 101909722 A | 08-12-2010 |
| | | EA 201001116 A1 | 28-02-2011 |
| | | EP 2234697 A1 | 06-10-2010 |
| | | US 2011009684 A1 | 13-01-2011 |
| | | WO 2009087155 A1 | 16-07-2009 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017207424 A, Stark
- US 20190381449 A, Liu
- US 20120310018 A, Lai
- US 6361582 B, Pinnau

**Non-patent literature cited in the description**

- **BAKER, RICHARD W.** Membrane Technology and Applications. McGraw-Hill, 2000, 290-291